# EUROPÄISCHE PATENTSCHRIFT

(11) **EP 3 269 341 B1**
(45) Veröffentlichungstag und Bekanntmachung des Hinweises auf die Patenterteilung: **08.09.2021**
(21) Anmeldenummer: 17181301.7
(22) Anmeldetag: 13.07.2017
(51) Int. Cl.: A61F 13/08

(54) **KOMPRESSIONSSTRUMPF MIT VERBESSERTER GEBRAUCHSTAUGLICHKEIT**
COMPRESSION STOCKINGS WITH IMPROVED USABILITY
BAS DE COMPRESSION AVEC LA FACILITÉ D'UTILISATION AMÉLIORÉE

(30) Priorität: 14.07.2016 DE 202016103817 U
(43) Veröffentlichungstag der Anmeldung: 17.01.2018
(73) Patentinhaber: Armbrust, Berthold, 64546 Mörfelden-Walldorf (DE); Armbrust-Andrzejewska, Jadwiga, 64546 Mörfelden-Walldorf (DE)
(72) Erfinder: FRANZ, Lotte, verstorben (DE)
(74) Vertreter: Meyer-Dulheuer MD Legal Patentanwälte PartG mbB

(56) Entgegenhaltungen:
- WO-A1-99/53876
- WO-A1-2014/116497
- DE-A1- 3 633 598
- DE-A1-102008 026 868
- US-A- 5 906 206

## Beschreibung

Die Erfindung betrifft einen Kompressionsstrumpf, der eine verbesserte Gebrauchstauglichkeit aufweist. Bekannte Kompressionsstrümpfe lassen sich nur beschwerlich an- und ausziehen. Der erfindungsgemäße Kompressionsstrumpf ist dazu ausgebildet, ein Bein von einem Bereich über dem Knie bis zu den Fußzehen zu umschließen. Gleichzeitig besitzt er einen oder zwei integrierte Reißverschlüsse. Diese erleichtern das An- und Ausziehen des Kompressionsstrumpfs. Der oder die Reißverschlüsse sind vorzugsweise im Kniebereich des Kompressionsstrumpfes angeordnet.

Kompressions- und Stützstrümpfe sind aus dem Stand der Technik bekannt. Sie werden insbesondere bei chronischen Venenleiden eingesetzt oder auch bei akuten Problemen wie einer Beinvenenthrombose. Bei solchen Leiden ist die Venenaktivität eingeschränkt, so dass ein erhöhter Druck in anderen Gefäßen im Bein entsteht. Dieser Druck wird bis in die Hautgefäße fortgeleitet. Durch Kompressionsstrümpfe wird von außen ein Gegendruck auf das Bein aufgebaut. Somit können sie die unmittelbaren Folgen einer Thrombose wie eine Schwellung lindern und außerdem die dauerhafte Schädigung von Gefäßen in den Beinen verhindern.

Ein Nachteil bekannter Stützstrümpfe liegt darin, dass diese sich nur sehr beschwerlich an- und ausziehen lassen. Dies liegt daran, dass Sie sehr eng am Bein anliegen müssen, um den gewünschten hohen Kompressionsdruck bewirken zu können. Häufig werden Stützstrümpfe von älteren Menschen benötigt, die teilweise nicht mehr die notwendige Kraft eigenständig aufbringen können, um die Stützstrümpfe selbstständig zu wechseln. Daher werden Lösungen gesucht, um die Gebrauchstauglichkeit von Stützstrümpfen zu verbessern.

Bekannt ist aus dem Deutschen Gebrauchsmuster DE 299 04 331 U1 ein aufblasbarer, druckregulierter Pneumatikstützstrumpf. Dieser verfügt über einen Klettverschluss und eine Luftkammer. Die Luftkammer ist zunächst nicht befüllt, wenn der Pneumatikstützstrumpf an das Bein angelegt wird. Er lässt sich somit leicht anlegen. Der Klettverschluss dient als Schließmechanismus. Anschließend wird die Luftkammer befüllt, sodass der Stützstrumpf den erwünschten Kompressionsdruck aufbaut. Ein Nachteil dieser Vorrichtung ist es jedoch, dass ihr Aufbau sehr kompliziert ist.

Aus dem Deutschen Gebrauchsmuster DE 87 07 838 U1 ist ein medizinischer Kompressionsstrumpf bekannt, der einen Klettverschluss besitzt. Auch hierdurch lässt sich ein leichtes An- und Ausziehen ermöglichen. Problematisch ist jedoch, dass ein Klettverschluss keine ausreichend große Verschlusskraft besitzt. So lässt sich mit einem solchen Verschluss kein ausreichend großer Druck auf das Bein aufbauen. Ferner besteht bei einem solchen Klettverschluss der Nachteil, dass er sich beim Laufen leicht öffnet.

Ferner sind aus dem Einzelhandel Kompressionsstrümpfe bekannt, die über einen Reißverschluss verfügen. Dieser erstreckt sich vom oberen Rand der Kompressionsstrümpfe in distaler Richtung, also von der Körpermitte weg, entlang des Kompressionsstrumpfes. Diese bekannten Kompressionsstrümpfe reichen jedoch nur zum Knie. Sie weisen somit keine umfassende Therapiewirkung betreffend das ganze Bein auf.

Aufgabe der vorliegenden Erfindung ist es deshalb, einen Kompressionsstrumpf mit umfassender Therapiewirkung bereitzustellen, der eine verbesserte Gebrauchstauglichkeit aufweist. Gelöst wird die Aufgabe durch einen Kompressionsstrumpf mit einem oder zwei integrierten Reißverschlüssen, der dazu ausgebildet ist, ein Bein von einem Bereich über dem Knie bis zu mindestens dem Fußknöchel zu umschließen. Die Form des Kompressionsstrumpfes ist zu diesem Zweck entsprechend angepasst. Somit ist eine Therapiewirkung bezüglich des gesamten Beins gewährleistet. Ferner birgt die Ausführung mit einem oder zwei Reißverschlüssen anstatt eines Klettverschlusses den Vorteil, dass ein unbeabsichtigtes Öffnen ausbleibt. Der Aufbau ist einfach, effektiv und preiswert in der Herstellung.

Der Kompressionsstrumpf ist aus einem Dehnmaterial gefertigt. Die Reißverschlüsse können aus Metall gefertigt werden, wobei es sich vorzugsweise um ein nicht allergenes Metall handeln sollte. Alternativ können die Reißverschlüsse auch aus Kunststoff gefertigt sein.

Erfindungsgemäß sind unterschiedliche Ausführungen der Reißverschlüsse denkbar. So ist gemäß einer ersten möglichen Ausführungsform der Erfindung ein einzelner Reißverschluss solchermaßen in den Kompressionsstrumpf eingebracht, dass er sich von einem Bereich, der sich bei Verwendung des Kompressionsstrumpfes oberhalb des Knies befindet, in axialer Richtung zu einem Bereich, der sich bei Verwendung des Kompressionsstrumpfes unterhalb des Knies befindet, erstreckt. In einer hierzu alternativen Ausführungsform der Erfindung können statt eines einzelnen durchgängigen Reißverschlusses auch zwei getrennte Reißverschlüsse verwendet werden. Ein solcher Aufbau sieht den oder die Reißverschlüsse in einem großen Bereich ober- und unterhalb des Knies vor. Dies erlaubt ein besonders leichtes An- und Ausziehen.

Gemäß einer weiteren Ausführungsform der Erfindung erstreckt sich der Reißverschluss von einem Bereich oberhalb des Knies bis hin zu einem Bereich auf Kniehöhe. Dies kann dann vorteilhaft sein, wenn sich für den Anwender keinerlei Probleme beim Anziehen des Kompressionsstrumpfes bis zur Kniehöhe ergeben.

Bei allen zuvor genannten Ausführungsformen kann der Bereich oberhalb des Knies, an dem der Reißverschluss endet, sich entweder im Dehnmaterial befinden oder aber durch den oberen Rand des Kompressionsstrumpfes definiert werden. Endet der Reißverschluss erst am oberen Rand des Kompressionsstrumpfes, so wird ein möglichst leichter Einstieg am oberen Ende des Kompressionsstrumpfes ermöglicht.

Außerdem ist es möglich, dass der Reißverschluss solchermaßen in den Kompressionsstrumpf eingebracht ist, dass er sich von einem Bereich, der sich bei Verwendung des Kompressionsstrumpfes unterhalb des Knies befindet, in axialer Richtung zu einem Bereich, der sich bei Verwendung des Kompressionsstrumpfes auf Kniehöhe befindet, erstreckt. Eine solche Ausführungsform kann dann vorteilhaft sein, wenn hauptsächlich der Unterschenkelbereich des Kompressionsstrumpfes beim An- und Ausziehen Probleme bereitet.

Der Reißverschluss verfügt gemäß einer möglichen Ausführungsform der vorliegenden Erfindung über einen einzelnen Schieber. Wenn ein Reißverschluss vorliegt, der sich über und unter dem Kniebereich erstreckt, so verfügt dieser vorzugsweise über zwei Schieber. Hierbei handelt es sich um einen distalen Schieber zum Einsatz unterhalb des Knies sowie einen proximalen Schieber zum Einsatz oberhalb des Knies. Das Vorhandensein mehrerer Schieber erleichtert das Öffnen und Schließen des Reißverschlusses. Es ist ferner möglich, dass der Reißverschluss mehr als zwei Schieber aufweist.

Die Reißverschlüsse sind an ihren Enden jeweils mit einem Anschlag versehen, der das Separieren der Reißverschlusshälften verhindert. Ist der Kompressionsstrumpf nur mit einem Reißverschluss ausgestattet, kann dieser zusätzlich einen Anschlag im Kniebereich vorsehen. Dieser mittlere Anschlag verhindert, dass ein Schieber über den Kniebereich hinaus bewegt wird. Der Anschlag sollte im Kniebereich so angebracht sein, dass er beim Abwinkeln des Knies für den Träger des Kompressionsstrumpfes keine Probleme bereitet. Es ist jedoch ebenso möglich, den Reißverschluss mit zwei oder mehr Schiebern ohne einen mittleren Anschlag zu verwenden.

Es ist besonders bevorzugt, wenn das Dehnmaterial zumindest zum Teil aus synthetischen Fasern hergestellt ist. Vorzugsweise handelt es sich bei dem Dehnmaterial um Vollsynthetikfasern, die eine besondere Kompressionskraft auszuüben vermögen. Jedoch sind erfindungsgemäß auch andere Dehnmaterialien einsetzbar.

Am distalen Ende des Dehnmaterials dieser Ausführungsform schließt sich ein zusätzliches Fasermaterial an, das dazu ausgebildet ist, die Fußzehen einzeln zu umschließen. Ein solcher Aufbau des Kompressionsstrumpfes dient dazu, die Fußzehen zu wärmen. Vorteilhaft ist hierbei, dass die Fußzehen trotzdem in einem besseren Kontakt mit dem Fußboden stehen, als dies durch den Kompressionsstrumpf hindurch der Fall wäre. Das zusätzliche Fasermaterial kann beispielsweise aus Baumwollmaterial hergestellt sein. Alternativ können als zusätzliches Fasermaterial aber auch Kunstfasern oder ein Verbundmaterial aus Baumwollmaterial und Kunstfasern verwendet werden.

Alternativ ist auch eine Ausführungsform der Erfindung möglich, gemäß der der Kompressionsstrumpf das Bein von einem Bereich über dem Knie bis über den kompletten Fuß umschließt.

Bei sämtlichen erfindungsgemäßen Ausführungsvarianten des Kompressionsstrumpfs ist es vorteilhaft, wenn das Dehnmaterial im Bereich, der sich beim Tragen des Kompressionsstrumpfs im Bereich der Kniescheibe befindet, eine Knieöffnung enthält. Dadurch wird ein Druck auf die Kniescheibe vermieden und die Kniescheibe, die durch das Dehnmaterial herausragt, kann sich beim Gehen ungehindert natürlich bewegen. Vorzugsweise ist dabei der Bereich des Dehnmaterials, welcher in der Kniekehle zu liegen kommt, so verarbeitet, dass beim Tragen keine Faltenbildung entsteht, wie dies bei der Anwendung herkömmlicher Kompressionsstrümpfe üblich ist und erfahrungsgemäß zu schmerzhaften Zuständen und teilweise sogar zu Entstehung von Hämatomen in diesem Bereich führt.

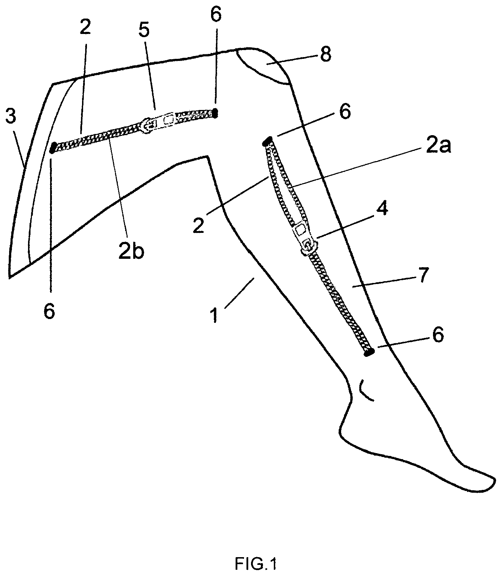

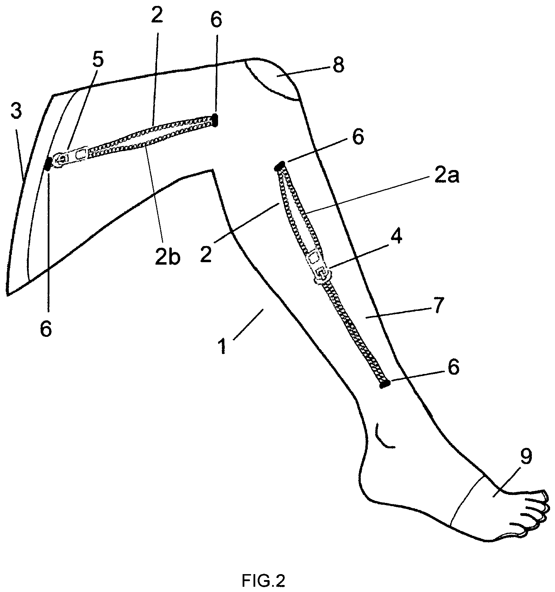

Die Erfindung ist in den beigefügten Zeichnungen dargestellt. Hierbei zeigt Fig. 1 eine erste mögliche Ausführungsform des erfindungsgemäßen Kompressionsstrumpfs **(1).** Fig. 2 zeigt eine zweite mögliche Ausführungsform des Kompressionsstrumpfes **(1).**

Fig. 1 zeigt eine erste mögliche Ausführungsform des erfindungsgemäßen Kompressionsstrumpfes **(1),** wobei der Kompressionsstrumpf **(1)** dazu ausgebildet ist, ein Bein vollständig zu umschließen. In den Kompressionsstrumpf **(1)** sind zwei Reißverschlüsse **(2)** integriert, die sich einmal von einem Bereich oberhalb des Knies zum Knie und einmal vom Knie zu einem Bereich unterhalb des Knies in Längsrichtung entlang des Kompressionsstrumpfes **(1)** erstrecken. Der distale Reißverschluss **(2a)** verfügt über einen distalen Schieber **(4)** und der proximale Reißverschluss **(2b)** über einen proximalen Schieber **(5).** Durch diese lassen sich die Reißverschlüsse **(2)** öffnen und schließen. An beiden Enden der Reißverschlüsse **(2)** befindet sich jeweils ein Anschlag **(6),** der den Bewegungsbereich von distalem Schieber **(4)** und proximalem Schieber **(5)** begrenzt. Der Kompressionsstrumpf **(1)** ist aus einem Dehnmaterial **(7)** auf Basis von Vollsynthetikfasern gefertigt und mit einer Knieöffnung **(8)** versehen, die die Kniescheibe beim Tragen frei lässt.

Fig. 2 zeigt eine zweite mögliche Ausführungsform des erfindungsgemäßen Kompressionsstrumpfes **(1).** In den Kompressionsstrumpf **(1)** sind zwei Reißverschlüsse **(2)** integriert, die sich einmal vom oberen Rand **(3)** des Kompressionsstrumpfes zum Knie und einmal vom Knie zu einem Bereich unterhalb des Knies in Längsrichtung entlang des Kompressionsstrumpfes **(1)** erstrecken. Der distale Reißverschluss **(2a)** verfügt über einen distalen Schieber **(4)** und der proximale Reißverschluss **(2b)** über einen proximalen Schieber **(5).** Durch diese lassen sich die Reißverschlüsse **(2)** öffnen und schließen. An beiden Enden der Reißverschlüsse **(2)** befindet sich im Reißverschluss **(2)** jeweils ein Anschlag **(6),** der den Bewegungsbereich von distalem Schieber **(4)** und proximalem Schieber **(5)** begrenzt. Ein erster Abschnitt des Kompressionsstrumpfes **(1)** ist aus einem Dehnmaterial **(7)** auf Basis von Vollsynthetikfasern gefertigt. Dieses umschließt das Bein bis zum Beginn der Fußzehen und ist mit einer Knieöffnung **(8)** versehen, die die Kniescheibe beim Tragen frei lässt. Ein zweiter Abschnitt des Kompressionsstrumpfes **(1)** besteht aus einem zusätzlichen Fasermaterial **(9),** das dazu ausgebildet ist, Fußzehen einzeln anliegend zu umschließen. Das zusätzliche Fasermaterial **(9)** ist bei dieser Ausführungsform aus Baumwolle gefertigt.

### Bezugszeichenliste

- 1: Kompressionsstrumpf
- 2: Reißverschluss
- 2a: Distaler Reißverschluss
- 2b: Proximaler Reißverschluss
- 3: Oberer Rand des Kompressionsstrumpfs
- 4: Distaler Schieber
- 5: Proximaler Schieber
- 6: Anschlag
- 7: Dehnmaterial
- 8: Knieöffnung
- 9: Zusätzliches Fasermaterial

## Patentansprüche

1. Kompressionsstrumpf (1) mit einem oder zwei integrierten Reißverschlüssen (2), wobei der Kompressionsstrumpf (1) dazu ausgebildet ist, ein Bein von einem Bereich über dem Knie bis zu mindestens dem Fußknöchel zu umschließen, **dadurch gekennzeichnet, dass**
• zwei Reißverschlüsse (2) solchermaßen in den Kompressionsstrumpf (1) eingebracht sind, dass ein Reißverschluss (2) sich von einem Bereich, der sich bei Verwendung des Kompressionsstrumpfes (1) oberhalb des Knies befindet, in axialer Richtung zu einem Bereich, der sich bei Verwendung des Kompressionsstrumpfes (1) auf Kniehöhe befindet, erstreckt und der andere Reißverschluss (2) sich von einem Bereich, der sich bei Verwendung des Kompressionsstrumpfes (1) auf Kniehöhe befindet, in axialer Richtung zu einem Bereich, der sich bei Verwendung des Kompressionsstrumpfes (1) unterhalb des Knies befindet, erstreckt oder ein Reißverschluss (2) solchermaßen in den Kompressionsstrumpf (1) eingebracht ist, dass er sich von einem Bereich, der sich bei Verwendung des Kompressionsstrumpfes (1) oberhalb des Knies befindet, in axialer Richtung zu einem Bereich, der sich bei Verwendung des Kompressionsstrumpfes (1) unterhalb des Knies befindet, erstreckt, und
• sich an das distale Ende des Dehnmaterials (7) ein zusätzliches Fasermaterial (9) anschließt, das dazu ausgebildet ist, Fußzehen einzeln anliegend zu umschließen.

2. Kompressionsstrumpf (1) nach Anspruch 1, **dadurch gekennzeichnet, dass** der Reißverschluss (2) solchermaßen in den Kompressionsstrumpf (1) eingebracht ist, dass er sich von einem Bereich, der sich bei Verwendung des Kompressionsstrumpfes (1) oberhalb des Knies befindet, in axialer Richtung zu einem Bereich, der sich bei Verwendung des Kompressionsstrumpfes (1) auf Kniehöhe befindet, erstreckt.

3. Kompressionsstrumpf (1) nach Anspruch 2, **dadurch gekennzeichnet, dass** der Bereich, der sich bei Verwendung des Kompressionsstrumpfes (1) oberhalb des Knies befindet, durch den oberen Rand (3) des Kompressionsstrumpfes (1) definiert wird.

4. Kompressionsstrumpf (1) nach Anspruch 1, **dadurch gekennzeichnet, dass** der Reißverschluss (2) solchermaßen in den Kompressionsstrumpf (1) eingebracht ist, dass er sich von einem Bereich, der sich bei Verwendung des Kompressionsstrumpfes (1) unterhalb des Knies befindet, in axialer Richtung zu einem Bereich, der sich bei Verwendung des Kompressionsstrumpfes (1) auf Kniehöhe befindet, erstreckt.

5. Kompressionsstrumpf (1) nach einem der Ansprüche 1 oder 3 mit Rückbezug auf Anspruch 1, **dadurch gekennzeichnet, dass** der Reißverschluss (2) einen distalen Schieber (4) und einen proximalen Schieber (5) umfasst.

6. Kompressionsstrumpf (1) nach Anspruch 5, **dadurch gekennzeichnet, dass**, wenn nur ein Reißverschluss (2) vorhanden ist, der Reißverschluss (2) einen Anschlag (6) für den distalen Schieber (4) und den proximalen Schieber (5) an einer Stelle, die sich beim Einsatz des Kompressionsstrumpfs (1) auf Höhe des Knies befindet, sowie je einen weiteren Anschlag (6) an beiden Enden des Reißverschlusses (2) aufweist oder, wenn zwei Reißverschlüsse (2a,2b) vorhanden sind, beide Reißverschlüsse (2a,2b) jeweils einen Anschlag (6) für die jeweiligen Schieber (4,5) an beiden Enden aufweisen.

7. Kompressionsstrumpf (1) nach einem der Ansprüche 5 oder 6, **dadurch gekennzeichnet, dass** der oder die Reißverschlüsse (2) so eingerichtet sind, dass eine Bewegung des distalen Schiebers (4) in distaler Richtung sowie des proximalen Schiebers (5) in proximaler Richtung zur Öffnung des Reißverschlusses (2) führt.

8. Kompressionsstrumpf (1) nach einem der vorhergehenden Ansprüche, **dadurch gekennzeichnet, dass** das Dehnmaterial (7) des Kompressionsstrumpfs (1) aus Vollsynthetikfasern hergestellt ist.

9. Kompressionsstrumpf (1) nach einem der vorhergehenden Ansprüche, **dadurch gekennzeichnet, dass** der Kompressionsstrumpf (1) dazu ausgebildet ist, ein Bein von einem Bereich über dem Knie bis zum Beginn der Fußzehen durch ein Dehnmaterial (7) zu umschließen.

10. Kompressionsstrumpf (1) nach einem der Ansprüche 1 bis 8, **dadurch gekennzeichnet, dass** das Dehnmaterial (7) des Kompressionsstrumpfs (1) dazu ausgebildet ist, ein Bein von einem Bereich über dem Knie bis über den kompletten Fuß zu umschließen.

11. Kompressionsstrumpf (1) nach einem der vorhergehenden Ansprüche, **dadurch gekennzeichnet, dass** das Dehnmaterial (7) im Bereich, der sich beim Tragen des Kompressionsstrumpfs (1) im Bereich der Kniescheibe befindet, eine Knieöffnung (8) enthält.

12. Kompressionsstrumpf (1) nach einem der vorhergehenden Ansprüche, **dadurch gekennzeichnet, dass** das zusätzliche Fasermaterial (9) an dem distale Ende des Dehnmaterials (7) aus Baumwollmaterial hergestellt ist.

13. Kompressionsstrumpf (1) nach einem der Ansprüche 1 bis 11, **dadurch gekennzeichnet, dass** das zusätzliche Fasermaterial (9) an dem distalen Ende des Dehnmaterials (7) aus Kunstfasern oder einem Verbundmaterial aus Baumwollmaterial und Kunstfasern hergestellt ist.

## Claims

1. A compression stocking (1) comprising one or two integrated zippers (2), wherein the compression stocking (1) is formed to surround a leg from a region above the knee all the way to at least the ankle, **characterized in that**
• two zippers (2) are introduced into the compression stocking (1) in such a way that a zipper (2) extends in the axial direction from a region located above the knee when using the compression stocking (1) to a region located at knee level when using the compression stocking (1), and the other zipper (2) extends in the axial direction from a region located at knee level when using the compression stocking (1) to a region located below the knee when using the compression stocking (1), or a zipper (2) is introduced into the compression stocking (1) in such a way that it extends in the axial direction from a region located above the knee when using the compression stocking (1) to a region located below the knee when using the compression stocking (1), and
• an additional fiber material (9), which is formed to surround toes so as to fit individually, adjoins the distal end of the stretch material (7).

2. The compression stocking (1) according to claim 1, **characterized in that** the zipper (2) is introduced into the compression stocking (1) in such a way that it extends in the axial direction from a region located above the knee when using the compression stocking (1) to a region located at knee level when using the compression stocking (1).

3. The compression stocking (1) according to claim 2, **characterized in that** the region located above the knee when using the compression stocking (1) is defined by the upper edge (3) of the compression stocking (1).

4. The compression stocking (1) according to claim 1, **characterized in that** the zipper (2) is introduced into the compression stocking (1) in such a way that it extends in the axial direction from a region located below the knee when using the compression stocking (1), to a region located at knee level when using the compression stocking (1).

5. The compression stocking (1) according to any one of claims 1 or 3, when dependent on claim 1, **characterized in that** the zipper (2) comprises a distal slider (4) and a proximal slider (5).

6. The compression stocking (1) according to claim 5, **characterized in that** when only one zipper (2) is present, the zipper (2) has a stop (6) for the distal slider (4) and the proximal slider (5) at a point located at knee level when using the compression stocking (1), as well as a respective further stop (6) at both ends of the zipper (2), or, when two zippers (2a, 2b) are present, both zippers (2a, 2b) each have a stop (6) for the respective sliders (4, 5) at both ends.

7. The compression stocking (1) according to any one of claims 5 or 6, **characterized in that** the zipper or the zippers (2) are set up such that a movement of the distal slider (4) leads in the distal direction, and of the proximal slider (5) in the proximal direction for opening the zipper (2).

8. The compression stocking (1) according to any one of the preceding claims, **characterized in that** the stretch material (7) of the compression stocking (1) is made of fully synthetic fibers.

9. The compression stocking (1) according to any one of the preceding claims, **characterized in that** the compression stocking (1) are formed to surround a leg from a region above the knee as far as the beginning of the toes by means of a stretch material (7).

10. The compression stocking (1) according to any one of claims 1 to 8, **characterized in that** the stretch material (7) of the compression stocking (1) is formed to surround a leg from a region above the knee over the entire foot.

11. The compression stocking (1) according to any one of the preceding claims, **characterized in that** the stretch material (7) includes a knee opening (8) in the region located in the region of the kneecap when wearing the compression stocking (1).

12. The compression stocking (1) according to any one of the preceding claims, **characterized in that** the additional fiber material (9) at the distal end of the stretch material (7) is made of cotton material.

13. The compression stocking (1) according to any one of claims 1 to 11, **characterized in that** the additional fiber material (9) at the distal end of the stretch material (7) is made of synthetic fibers or a composite material of cotton material and synthetic fibers.

## Revendications

1. Bas de compression (1) avec une ou deux fermetures éclair (2) intégrées, le bas de compression (1) étant conçu pour envelopper une jambe depuis une zone au-dessus du genou jusqu'à au moins la cheville, **caractérisé en ce que**
• deux fermetures éclair (2) sont intégrées dans le bas de compression (1) de telle manière à ce qu'une fermeture éclair (2) s'étende depuis une zone qui se trouve au-dessus du genou lorsque le bas de compression (1) est porté, dans le sens axial vers une zone qui se trouve à hauteur du genou lorsque le bas de compression (1) est porté, et que l'autre fermeture éclair (2) s'étende depuis une zone qui se trouve à hauteur du genou lorsque le bas de compression (1) est porté, dans le sens axial vers une zone qui se trouve en dessous du genou lorsque le bas de compression (1) est porté, ou une fermeture éclair (2) est intégrée dans le bas de compression (1) de telle manière à s'étendre depuis une zone qui se trouve au-dessus du genou lorsque le bas de compression (1) est porté, dans le sens axial vers une zone qui se trouve en dessous du genou lorsque le bas de compression (1) est porté, et **en ce que**
• une matière en fibres (9) supplémentaire, conçue pour envelopper étroitement chacun des orteils, est reliée à l'extrémité distale de la matière extensible (7).

2. Bas de compression (1) conformément à la revendication 1, **caractérisé en ce que** la fermeture éclair (2) est intégrée dans le bas de compression (1) de telle manière à s'étendre depuis une zone qui se trouve au-dessus du genou lorsque le bas de compression (1) est porté, dans le sens axial vers une zone qui se trouve à hauteur du genou lorsque le bas de compression (1) est porté.

3. Bas de compression (1) conformément à la revendication 2, **caractérisé en ce que** la zone qui se trouve au-dessus du genou lorsque le bas de compression (1) est porté, est définie par le bord supérieur (3) du bas de compression (1).

4. Bas de compression (1) conformément à la revendication 1, **caractérisé en ce que** la fermeture éclair (2) est intégrée dans le bas de compression (1) de telle manière à s'étendre depuis une zone qui se trouve en dessous du genou lorsque le bas de compression (1) est porté, dans le sens axial vers une zone qui se trouve à hauteur du genou lorsque le bas de compression (1) est porté.

5. Bas de compression (1) conformément à l'une des revendications 1 ou 3 avec référence à la revendication 1, **caractérisé en ce que** la fermeture éclair (2) comprend une glissière distale (4) et une glissière proximale (5).

6. Bas de compression (1) conformément à la revendication 5, **caractérisé en ce qu'**une seule fermeture éclair (2) est existante, la fermeture éclair (2) présentant une butée (6) pour la glissière distale (4) et la glissière proximale (5) à un endroit qui se trouve à hauteur du genou lorsque le bas de compression (1) est porté, ainsi qu'une autre butée (6) aux deux extrémités de la fermeture éclair (2) ou, si deux fermetures éclair (2a, 2b) sont existantes, les deux fermetures éclair (2a, 2b) présentant une butée (6) pour chacune des glissières (4, 5) aux deux extrémités.

7. Bas de compression (1) conformément à l'une des revendications 5 ou 6, **caractérisé en ce que** la ou les fermetures éclair (2) sont mises en place de telle manière à ce qu'un mouvement de la glissière distale (4) dans le sens distal et de la glissière proximale (5) dans le sens proximal conduise à l'ouverture de la fermeture éclair (2).

8. Bas de compression (1) conformément à l'une des revendications précédentes, **caractérisé en ce que** la matière extensible (7) du bas de compression (1) est réalisée en fibres entièrement synthétiques.

9. Bas de compression (1) conformément à l'une des revendications précédentes, **caractérisé en ce que** le bas de compression (1) est conçu pour envelopper une jambe depuis une zone au-dessus du genou jusqu'au début des orteils par une matière extensible (7).

10. Bas de compression (1) conformément à l'une des revendications 1 à 8, **caractérisé en ce que** la matière extensible (7) du bas de compression (1) est conçue pour envelopper une jambe depuis une zone au-dessus du genou jusqu'au-dessus du pied dans son ensemble.

11. Bas de compression (1) conformément à l'une des revendications précédentes, **caractérisé en ce que** la matière extensible (7) contient, dans la zone qui se trouve au niveau de la rotule lorsque le bas de compression (1) est porté, une ouverture pour le genou (8).

12. Bas de compression (1) conformément à l'une des revendications précédentes, **caractérisé en ce que** la matière en fibres (9) supplémentaire à l'extrémité distale de la matière extensible (7) est réalisée en coton.

13. Bas de compression (1) conformément à l'une des revendications 1 à 11, **caractérisé en ce que** la matière en fibres (9) supplémentaire à l'extrémité distale de la matière extensible (7) est réalisée en fibres synthétiques ou dans une matière composée de coton et de fibres synthétiques.
